# EUROPÄISCHE PATENTSCHRIFT

(11) **EP 1 142 084 B1**
(45) Veröffentlichungstag und Bekanntmachung des Hinweises auf die Patenterteilung: **17.08.2005**
(21) Anmeldenummer: 99962070.1
(22) Anmeldetag: 26.11.1999
(51) Int. Cl.: H02K 9/22, H02K 5/02, A61M 1/10

(54) **FLUIDGEKÜHLTER ELEKTROMOTOR MIT HOHER LEISTUNGSDICHTE**
ELECTRIC MOTOR COOLED BY A FLUID AND HAVING HIGH SPECIFIC POWER
MOTEUR ELECTRIQUE REFROIDI PAR UN LIQUIDE ET A PUISSANCE VOLUMIQUE ELEVEE

(30) Priorität: 02.12.1998 DE 29821564 U
(43) Veröffentlichungstag der Anmeldung: 10.10.2001
(73) Patentinhaber: Impella CardioSystems AG, 52074 Aachen (DE)
(72) Erfinder: SIESS, Thorsten, D-52146 Würselen (DE)
(74) Vertreter: Selting, Günther, Dipl.-Ing.
(86) Internationale Anmeldenummer: PCT/DE1999/003748
(87) Internationale Veröffentlichungsnummer: WO 2000/033446

(56) Entgegenhaltungen:
- EP-A- 0 581 966
- WO-A-98/44619
- US-A- 4 387 311
- US-A- 5 783 888
- PATENT ABSTRACTS OF JAPAN vol. 005, no. 030 (E-047), 24. Februar 1981 (1981-02-24) & JP 55 155530 A (MITSUBISHI ELECTRIC CORP), 3. Dezember 1980 (1980-12-03)

## Beschreibung

Die Erfindung betrifft einen fluidgekühlten Elektromotor mit hoher Leistungsdichte mit einem aus einem Polymermaterial gespritzten Motorgehäuse, welches einen Stator umgibt, und insbesondere einen Mikromotor, der in das Blutgefäßsystem eines Körpers eingeführt werden kann, um eine im Körper befindliche Blutpumpe anzutreiben.

In WO 98/44619, von der der Oberbegriff des Patentanspruchs 1 ausgeht, ist ein Mikromotor beschrieben, der Bestandteil einer in den Körper eines Patienten einzuführenden Blutpumpe ist. Der Mikromotor weist ein aus Polymermaterial gespritztes Motorgehäuse auf, dessen Außendurchmesser 8,0 mm nicht überschreitet. Der Motor ist Bestandteil einer Blutpumpe, die im Gefäßsystem eines Patienten Blut pumpt, das an dem Motorgehäuse entlangströmt und dadurch den Motor kühlt. Im Motorgehäuse entsteht eine relativ hohe Temperatur, da das Polymermaterial ein schlechter Wärmeleiter ist. Infolge des Thermoskannen-Effekts ergeben sich Temperaturen von 60 °C. Obwohl das Motorgehäuse durch das entlangströmende Blut, das eine Temperatur von 37 °C hat, gekühlt wird, hat die aus dem Motorgehäuse herausragende Welle eine höhere Temperatur. Es besteht die Gefahr, daß das temperaturkritische Medium Blut durch eine zu hohe Wellentemperatur beschädigt wird. Dichtungen, die eine hochtemperierte Welle abdichten, unterliegen einem erhöhten Verschleiß, wodurch die Lebensdauer des Mikromotors verringert wird. Ferner ist zu berücksichtigen, daß bei einer hohen Innentemperatur im Motor sich der Wirkungsgrad verschlechtert. Gerade bei Mikromotoren, die häufig auf Batteriebetrieb angewiesen sind, ist ein hoher Wirkungsgrad sehr wichtig. Durch eine zu hohe Innentemperatur im Motor und der damit einhergehenden Wirkungsgradverschlechterung wird auch die mechanisch abgreifbare Leistung reduziert.

US-A-4,387,311 beschreibt einen Motor, dessen Gehäuse wärmeverteilend ist und zu diesem Zweck verschiedene Füllstoffe enthält sowie Aluminiumhydroxidpulver. Das Aluminiumhydroxidpulver dient dazu, eine überschüssige Wärme, die von der Maschine erzeugt wird, wenn diese zwangsweise angehalten wird, abzuführen. Das Aluminiumhydroxid wird dann einem Kristallisationsprozess unterworfen, wobei Wärme verbraucht wird, bevor die Maschinenteile durch Pyrolyse zerstört werden. Das Aluminiumhydroxidpulver ist kein Material mit hoher Wärmeleitfähigkeit und dient auch nicht zur Ableitung von Wärme an die Umgebung. Auch die Füllstoffe haben nicht die Funktion der Wärmeableitung. Sie dienen der größeren Wirtschaftlichkeit.

US-A-5,783,888 beschreibt einen Motor, bei dem ein Teil, das aus synthetischem Kunstharz besteht, eine hohe Wärmeleitfähigkeit haben soll. Die Wärme wird beispielsweise durch den Außenumfang des Kunstharzteils an die Außenluft abgegeben, die durch Flügel an dem Kunstharzteil entlangbewegt wird. Ein Motorgehäuse, das den gesamten Motor umkapselt, ist in dieser Schrift nicht beschrieben.

Der Erfindung liegt die Aufgabe zugrunde, einen fluidgekühlten Mikromotor mit verbesserter Wärmeabfuhr zu schaffen.

Die Lösung dieser Aufgabe erfolgt erfindungsgemäß mit den im Patentanspruch 1 angegebenen Merkmalen. Bei dem erfindungsgemäßen Mikromotor enthält das Polymermaterial des Motorgehäuses einen wärmeleitfähigen elektrisch isolierenden Füllstoff. Dieser Füllstoff bewirkt, daß das Motorgehäuse eine hohe Leitfähigkeit erhält, so daß die im Gehäuseinneren entstehende Wärme besser an das umgebende Blut abgeführt werden kann. Der Füllstoff besteht vorzugsweise aus einem Keramikmaterial, insbesondere Al₂O₃. Durch dieses Material wird bei entsprechendem Füllanteil die Wärmeleitfähigkeit von Thermo- oder Duroplasten signifikant von typischerweise 0,05 W/mK auf bis zu 2 W/mK erhöht, ohne daß die elektrische Leitfähigkeit zunimmt. Es hat sich gezeigt, daß bei einer Fluidtemperatur von 37 °C die Motorinnentemperatur von ursprünglich 60 °C auf 40 °C bis 45 °C verringert werden kann. Im Motorinnern entsteht also kein Wärmestau, weil durch das wärmeleitfähige Motorgehäuse die Wärme besser an das Blut abgeführt wird. Dadurch sind die Komponenten des Elektromotors einer geringeren thermischen Belastung und somit auch einem geringeren Verschleiß unterworfen. Ferner kann der Motor mit einem verbesserten Wirkungsgrad betrieben werden.

Der Füllstoffanteil beträgt mindestens 40 Gewichtsprozent, um eine signifikante Steigerung der Wärmeleitfähigkeit des Matrixmaterials zu erzielen. Der Füllstoff, der ursprünglich als feines Pulver vorliegt, wird in das flüssige Polymermaterial eingemischt und anschließend erfolgt die Formgebung im Spritzgussverfahren, Tränkungsverfahren u.dgl.

Das Motorgehäuse hat eine Länge, die mindestens das Zweifache des Außendurchmessers dieses Gehäuses beträgt. Es handelt sich also um ein langgestrecktes Motorgehäuse von kleinem Durchmesser. Ein solches Motorgehäuse hat eine im Verhältnis zum Volumen große Oberfläche. Durch den in der Gehäusewand enthaltenen wärmeleitfähigen Füllstoff wird in Verbindung mit der großen Gehäuseoberfläche eine wirksame Wärmeableitung in ein an dem Gehäuse entlangströmendes Medium erreicht. Vorzugsweise beträgt die Länge des Motorgehäuses mindestens das 2,5-fache, insbesondere mindestens das Dreifache, des Außendurchmessers.

Der Füllstoff hat auch Vorteile bei der Herstellung des Mikromotors im Spritzgußverfahren oder im Vakuumgußverfahren, weil bei einem hohen Füllstoffanteil die Aushärtezeit eines Duromers aufgrund der geringeren Duromerdicken und der besseren Wärmeleitfähigkeit verringert wird. Dadurch wird die Warmaushärtung im Ofen wesentlich verkürzt.

Einen wesentlichen Isolator im Innern eines Mikromotors bilden die Drahtwicklungen. Zwischen den Windungen bestehen mit Luft gefüllte Hohlräume, die einen idealen Wärmeisolator bilden. Außerdem sind die einzelnen Drahtwindungen mit Isoliermänteln umgeben, die ebenfalls gute thermische Isolatoren bilden. Gemäß einer bevorzugten Weiterbildung der Erfindung, die selbständige Bedeutung hat, sind die Hohlräume der Wicklungen des Stators mit einem einen wärmeleitfähigen Füllstoff enthaltenden Polymermaterial aufgefüllt. Dadurch werden die Wicklungen zu guten Wärmeleitern, wodurch die in den Wicklungen entstandene strominduzierte Wärme mit geringem Wärmewiderstand an den Rückschluß und/oder das Motorgehäuse abgeleitet wird. Das die Hohlräume ausfüllende Polymermaterial bewirkt eine verbesserte Wärmeabgabe an die Rückschlußblechanordnung, die dann ihrerseits die Wärme an das Motorgehäuse weiterleitet. Die Einbringung des mit Füllstoff versehenen Polymermaterials in die Hohlräume der Wicklungen erfolgt zweckmäßigerweise durch Tränkung oder auch durch einen Vergußprozeß im Zusammenwirken von Vakuum und Druck, bei dem sämtliche Hohlräume gezielt mit wärmeleitfähigem Polymermaterial gefüllt werden.

Im folgenden wird unter Bezugnahme auf die Zeichnungen ein Ausführungsbeispiel der Erfindung näher erläutert.

Es zeigen:
- Fig. 1: einen Längsschnitt durch den Mikromotor und die damit verbundene Pumpe, und
- Fig. 2: in vergrößertem Maßstab die Darstellung der Einzelheit II aus Fig. 1.

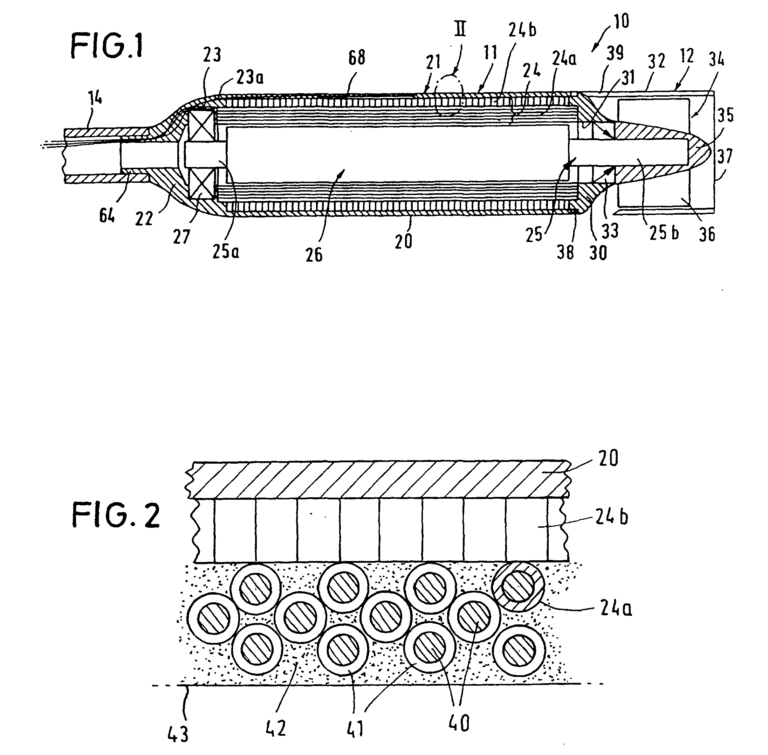

Die nachstehend beschriebene Fig. 1 ist bereits Gegenstand von WO 98/44619:

In Fig. 1 ist eine intravasale Blutpumpe 10 dargestellt, also eine Blutpumpe, die durch das Blutgefäßsystem eines Patienten geschoben werden kann, um bis in das Herz hinein vorzudringen Der Außendurchmesser einer solchen Blutpumpe ist in der Regel an keiner Stelle größer als 8 mm.

Die Pumpe 10 weist einen Antriebsteil 11 und einen damit stark verbundenen Pumpenteil 12 auf. Der Antriebsteil 11 enthält einen elektrischen Mikromotor 21 mit einem langgestreckten zylindrischen Gehäuse 20. An dem rückwärtigen Ende ist das Gehäuse 20 mit einer Stirnwand 22 verschlossen, an die sich ein flexibler Katheter 14 abdichtend anschließt. Durch den Katheter 14 verlaufen die elektrischen Kabel 23 zur Stromversorgung und zur Steuerung des Elektromotors 21, und außerdem weitere Kabel 23a, die mit Sensoren der Blutpumpe 10 verbunden sind.

Der Stator 24 des Motors weist in üblicher Weise zahlreiche umfangsmäßig verteilt angeordnete Wicklungen 24a sowie einen aus Blechen bestehenden magnetischen Rückschluß 24b in Längsrichtung auf. Er ist mit dem Motorgehäuse 20 umspritzt. Der Stator 24 umgibt den mit der Motorwelle 25 verbundenen Rotor 26, der aus in Wirkrichtung magnetisierten Permanentmagneten besteht. Die Motorwelle ist am rückwärtigen Ende mit einem Lager 27 im Motorgehäuse bzw. in der Stirnwand 22 gelagert. Die Welle erstreckt sich durch die gesamte Länge des Motorgehäuses 20 und ragt nach vorne aus diesem heraus.

Den vorderen Abschluß des Motorgehäuses bildet ein rohrförmiges stationäres Nabenteil 30, das integraler Bestandteil des Gehäuses 20 ist. Der Außendurchmesser des Nabenteils verjüngt sich zum vorderen Ende hin, wo sich ein Lager 33 zur Lagerung der Motorwelle 25 befindet. Dieses Lager ist zugleich als Wellendichtung ausgebildet.

Die Motorwelle 25 steht aus dem Nabenteil 30 nach vorne vor und trägt dort ein Flügelrad 34 mit einer auf dem Wellenende sitzenden Nabe 35 und davon abstehenden Flügeln 36 oder Pumpenschaufeln, die in bezug auf die Achse des Flügelrades 34 schräggestellt sind. Das Flügelrad 34 ist in einem zylindrischen Pumpengehäuse 32 enthalten, das durch drei umfangsmäßig verteilte Stege 39 mit einem Ring 38 verbunden ist, welcher auf dem Nabenteil 30 sitzt. Man erkennt, daß das Motorgehäuse 20 und das Pumpengehäuse 32 starr miteinander verbunden sind und gleiche Außendurchmeser haben, und daß der Durchmesser der Pumpe 10 an keiner Stelle größer ist als dieser Außendurchmesser.

Bei einer Rotation des Flügelrades 34 wird Blut durch die Ansaugöffnung 37 des Pumpengehäuses 32 angesaugt und in axialer Richtung im Pumpengehäuse 32 nach hinten getrieben. Durch den ringförmigen Spalt zwischen dem Pumpengehäuse 32 und dem Motorgehäuse 20 strömt das Blut am Nabenteil 30 entlang nach außen, um weiter an dem Motorgehäuse 20 entlangzuströmen. Hierdurch wird der Abtransport der im Antrieb erzeugten Wärme sichergestellt, ohne daß es zur Blutschädigung durch zu hohe Oberflächentemperaturen (über 41 °C) auf dem Motorgehäuse 20 kommt.

Es ist auch möglich, den Pumpenteil 12 mit umgekehrter Förderrichtung zu betreiben, wobei das Blut an dem Motorgehäuse entlang angesaugt wird und aus der stirnseitigen Öffnung 37 axial austritt.

In die Umfangswand des Motorgehäuses 20 ist ein Drucksensor 68 eingebettet, der mit einer Leitung 23a in Verbindung steht. Diese Leitung 23a ist in dem Motorgehäuse 20 vergossen und sie führt durch die Stirnwand 22 hindurch in den Katheter 14. Am proximalen Katheterende sind die Leitung 23a und das Kabel 23 mit einem extrakorporalen Steuergerät verbindbar, das den Betrieb der Pumpe 10 steuert.

Die Herstellung des Mikromotors 21 erfolgt in einem Spritzgußverfahren mit einer Spritzgußform, in die ein Dorn eingebracht wird, welcher die Statorkomponenten 24a,24b trägt. Dabei wird Polymermaterial in die Spritzgußform injiziert. Anschließend werden Spritzgußform und Dorn von dem Motorgehäuse 20 entfernt und schließlich wird der Rotor 26 durch die Öffnung 31 des Nabenteils 30 hindurch montiert.

Das verwendete Polymermaterial ist vorzugsweise ein flüssiges Epoxydharz, das einen Füllstoffanteil von mindestens 40 Gewichtsprozent enthält. Als Füllstoff wird Al₂O₃ als feines Pulver benutzt.

Die in den Wicklungen 24a des Stators 24 entstehende strominduzierte Wärme wird zunächst an die Rückschlußanordnung 24b übertragen und von dieser dann an das Motorgehäuse 20 durch Wärmeleitung weitergegeben. Da das Motorgehäuse 20 eine hohe Wärmeleitfähigkeit hat, entsteht ein geringer Temperaturgradient zu dem das Motorgehäuse umströmenden Blut hin, dessen Temperatur 37 °C beträgt.

In Fig. 2 sind die Drahtwindungen 40 einer Wicklung 24a dargestellt, wobei jeder Draht mit einem Isoliermantel 41 versehen ist. Die Zwischenräume zwischen den Drahtwindungen bzw. deren Isolierungen sind mit Polymermaterial 42 ausgefüllt, welches Al₂O₃ als Füllstoff enthält. Auch hier beträgt der Füllstoffanteil mindestens 40 Gewichtsprozent. Das die Hohlräume ausfüllende Polymermaterial 42 steht mit der Rückschlußblechanordnung 24b in vollflächigem Kontakt, ebenso wie ein vollflächiger Kontakt der Rückschlußblechanordnung 24b mit dem Motorgehäuse 20 besteht.

Aufgrund der verbesserten Kühlung kann bei u = 30 000 U/min eine hohe mechanische Leistungsabgabe von 5 W erzielt werden. Aufgrund der deutlich verbesserten Wärmeleitfähigkeit in allen Motorkomponenten und der deutlich verbesserten Wärmeankopplung an den Übergängen zwischen Wicklung, Rückschluß und Motorgehäuse kann die Temperatur im Motorinnern signifikant reduziert werden.

So ist bei dem beschriebenen Aufbau für die Übertragung von 10 W Wärme aus dem Motorinnern nach außen nur noch eine treibende Temperaturdifferenz von Δϑ = 5-8 K gegenüber Δϑ = 18-25 K bei Verwendung eines ungefüllten Polymers notwendig.

Durch die vollständige Füllung aller Wicklungshohlräume mit Polymermaterial 42 kann zu dem auf der Wicklungsinnenseite ein glattflächiger und isolierender Wicklungsüberzug 43 erreicht werden. Im Falle der Ausführung des Motors als bürstenlose Synchronmaschine kann somit der Motor bei vollständiger Kapselung der Wicklung auch im Motorinneren flüssigkeitsgefüllt betrieben werden.

## Patentansprüche

1. Fluidgekühlter Elektromotor mit einem aus Polymermaterial geformten Motorgehäuse (20), welches einen Stator (24) umgibt, wobei das Motorgehäuse eine Länge hat, die mindestens das Zweifache des Außendurchmessers des Motorgehäuses beträgt,
**dadurch gekennzeichnet,**
**daß** das Polymermaterial des Motorgehäuses (20) einen wärmeleitfähigen elektrisch isolierenden Füllstoff mit einem Gewichtsanteil von mindestens 40 % enthält.

2. Elektromotor nach Anspruch 1, **dadurch gekennzeichnet, daß** der Füllstoff Al₂O₃ aufweist.

3. Elektromotor insbesondere nach Anspruch 1 oder 2, **dadurch gekennzeichnet, daß** der Stator (24) Wicklungen aufweist, deren Hohlräume mit einem einen wärmeleitfähigen Füllstoff enthaltenden Polymermaterial (42) aufgefüllt sind.

4. Elektromotor nach Anspruch 3, **dadurch gekennzeichnet, daß** das die Hohlräume auffüllende Polymermaterial (42) mit einer umgebenden Rückschlußblechanordnung (24b) in wärmeleitendem Kontakt steht.

5. Elektromotor nach einem der Ansprüche 1 bis 4, **dadurch gekennzeichnet, daß** ein von dem Motor angetriebener Pumpenteil (12) ebenfalls Füllstoff enthält.

6. Elektromotor nach Anspruch 3 oder 4, **dadurch gekennzeichnet, daß** die Wicklung eine an dem Polymermaterial bestehende glatte, isolierende Innenseite (43)aufweist.

## Claims

1. A fluid-cooled electric motor with a motor housing (20) made of polymer material and enclosing a stator (24), the length of the motor housing being at least twice the outer diameter of the motor housing,
**characterized in that**
the polymer material of the motor housing (20) contains a thermally conductive, electrically insulating filler of at least 40 percent by weight.

2. The electric motor of claim 1, wherein the filler comprises Al₂O₃.

3. The electric motor of claim 1 or 2, wherein the stator (24) comprises coils whose cavities are filled with a polymer material (42) including a thermally conductive filler.

4. The electric motor of claim 3, wherein the polymer material (42) filling the cavities is in thermally conductive contact with a surrounding yoke sheet arrangement (24b).

5. The electric motor of one of claims 1 to 4, wherein a pump portion (12) driven by the motor also includes filler.

6. The electric motor of claim 3 or 4, wherein the coil has a smooth insulating inner surface (43) made of said polymer material.

## Revendications

1. Moteur électrique refroidi par fluide comprenant un carter de moteur (20) en matériau polymère et entourant un stator (24), le carter de moteur présentant une longueur qui correspond au moins deux fois à son diamètre extérieur, **caractérisé en ce que** le matériau polymère du carter de moteur (20) contient une charge thermiquement conductrice électriquement isolante avec une proportion en poids d'au moins 40%.

2. Moteur électrique selon la revendication 1, **caractérisé en ce que** la charge présente de l'Al₂O₃.

3. Moteur électrique notamment selon la revendication 1 ou 2, **caractérisé en ce que** le stator (24) présente des enroulements dont les espaces creux sont remplis d'un matériau polymère (42) contenant une charge thermiquement conductrice.

4. Moteur électrique selon la revendication 3, **caractérisé en ce que** le matériau polymère (42) remplissant les espaces creux est en contact thermiquement conducteur avec un dispositif de tôles de réinjection (24b) entourant.

5. Moteur électrique selon l'une quelconque des revendications 1 à 4, **caractérisé en ce qu'**une partie pompe (12) entraînée par le moteur contient également une charge.

6. Moteur électrique selon la revendication 3 ou 4, **caractérisé en ce que** l'enroulement présente une face intérieure (43) lisse isolante sur le matériau polymère.
